Europäisches Patentamt

European Patent Office

Office européen des brevets

(11) Publication number: **0 039 556**
**B1**

(12) **EUROPEAN PATENT SPECIFICATION**

(45) Date of publication of patent specification: **16.01.85**

(21) Application number: **81301797.7**

(22) Date of filing: **23.04.81**

(51) Int. Cl.⁴: **C 07 C 76/02,** C 07 C 79/10,
C 07 C 87/52

(54) Water removal in a process for continuous nitration of a nitratable aromatic compound.

(30) Priority: **24.04.80 US 143372**

(43) Date of publication of application:
**11.11.81 Bulletin 81/45**

(45) Publication of the grant of the patent:
**16.01.85 Bulletin 85/03**

(84) Designated Contracting States:
**AT BE CH DE FR GB IT LI LU NL SE**

(56) References cited:
**DE-C- 971 523**
**SU-A- 18 154**
**US-A-3 928 475**
**US-A-3 957 889**

(73) Proprietor: **E.I. DU PONT DE NEMOURS AND
COMPANY
1007 Market Street
Wilmington Delaware 19898 (US)**

(72) Inventor: **MC Call, Robert
28 Timber Line Drive
Newark Delaware 19711 (US)**

(74) Representative: **Woodcraft, David Charles et al
BROOKES & MARTIN High Holborn House 52/54
High Holborn
London, WC1V 6SE (GB)**

Courier Press, Leamington Spa, England.

## Description

This invention relates to the mixed acid process for nitrating aromatic hydrocarbons and is more particularly directed to a procedure in that process for removing water from the reaction mass so as to maintain adequate sulfuric acid concentration.

The nitrogen of aromatic hydrocarbons, especially benzene, is of great commercial importance. Although many nitration processes are known, the one most widely used is continuous mixed acid nitration.

In that process, an aromatic hydrocarbon compound and nitric acid are brought together in a medium of concentrated sulfuric acid. The sulfuric acid acts on the nitric acid to form nitronium ions $(NO_2^+)$, the actual nitrating agent.

The rate of reaction in the process is governed by the concentration of nitronium ions in the medium, higher concentrations, of course, giving faster nitration. The concentration of nitronium ions, in turn, is goverened by the concentration of sulfuric acid in the medium. As the nitration proceeds, water is continuously brought into the medium with the nitric acid feed, and is also produced as a reaction product. This water dilutes the medium and lowers its sulfuric acid content eventually to the point where the nitration reaction slows, or even stops.

In practice therefore, it is necessary continuously to remove water from the reaction medium to keep the sulfuric acid content at its optimum concentration of about 70—75%, by weight, and thus maintain the reaction at maximum efficiency. The most common method of doing this is continuously to run the medium through a concentrator.

DE—C—971523 discloses vapour phase nitration in which a low-boiling reactant (benzene) is brought in vapour form to the site of reaction by means of an inert gas but the amount of gas used, taking into account other parameters, is incapable of achieving any realistic maintenance of sulfuric acid concentration in a reaction medium. USSR Patent 18.154 similarly discloses the contact of a nitration agent with an inert gas/reactant vapour mixture. At the reaction temperatures suggested the volume of gas required to achieve adequate water-removal would be enormous and would displace the reaction mass so as in practice not to result in intimate contact (and very little water might in fact be removed in these circumstances). "Methoden der organischen Chemie (Houben-Weyl)", 1/2, page 876, lines 36 and 37 (1959), Georg Thieme Verlag, Stuttgart discloses water-removal rom substances using dry inert gases.

From a practical standpoint, water-removal by continuously running reaction medium through a concentrator has in the past been regarded as generally satisfactory. However, recent emphasis has made it less desirable because the method generates sulfuric acid mist which must be dealt with, and because large amounts of energy are required. Besides this, the concentrators are expensive because they must be made of materials strong enough to withstand constant exposure to the strong acid.

It has now been found that water can be removed from such a nitration reaction medium by passing an inert gas through it according to the process defined in the claims. As the gas flows through the medium it becomes humidified and carries captured water with it as it leaves the reactor.

The principal advantages of this are:—

(1) most of the energy needed for humidification of the gas will normally come from the heat of the reaction itself;

(2) expensive extrinsic drying equipment is not needed; and

(3) air pollution in the form of sulfuric acid mist is minimized. According to the invention, there is provided a process for continuous nitration of an aromatic compound by the mixed acid method wherein the reaction mass is contacted with a gas inert to the nitration reaction and capable of being humidified under the reaction conditions, thereby removing water from the reaction mass, and withdrawing humidified inert gas from the reaction zone, characterized in that the inert gas is contacted intimately with the reaction mass by sparging in sufficient amount and at sufficient rate that water is removed from the reaction mass at a rate which maintains at least the minimum critical sulfuric acid concentration required for continuous nitration of the aromatic starting material.

Examples of aromatic hydrocarbons which can be nitrated according to the process of the invention are benzene, toluene and chlorobenzene. Benzene is of special interest, nitrobenzene being useful in particular as an intermediate in the preparation of aniline and other commodity chemicals.

The nitrogen reaction is conveniently effected at 120—140°C, preferably about 130°C. A pressure of about 3450 pascals (gauge) will conveniently be adopted.

The inert gas should obviously be inert to the nitration reaction, contain as little oxygen as possible, and be capable of being humidified under the reaction conditions. Nitrogen, halogenated hydrocarbons, carbon dioxide and the inert atmospheric gases are examples. Nitrogen is preferred.

The gas may be fed to the reaction at a temperature of 20—60°C, preferably about 40°C, and can be bubbled through the reaction medium at a rate of 130—150 moles per hour, preferably 145 moles per hour. The gas rises through the reaction mass, is humidified by water extracted from the mass, and can then be withdrawn from the reactor.

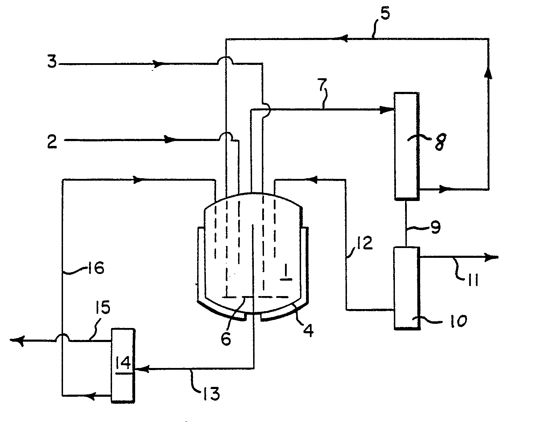

The invention will now be described with reference to the accompanying drawing which is a flow sheet representing schematically an apparatus and process for carrying out mixed acid nitration according to the invention.

Reactor 1 is charged with 60—70% sulfuric acid, and nitrated aromatic hydrocarbon of the kind to be produced, in an acid/hydrocarbon weight ratio of 50—60/40—50. Aromatic hydrocarbon and nitric acid (60—70%) are then continuously fed into the reactor through lines 2 and 3 to form a reaction mass having an aqueous phase and an organic phase.

The hydrocarbon and nitric acid are fed into the reactor at such rates and in such ratios to each other that, after reaction has begun, the aqueous phase contains, at any given time,

| | |
|---|---|
| sulfuric acid | 60—70%, by weight preferably about 65% |
| nitric acid | 0.1—0.3%, preferably 0.2% |
| water | a complementary amount |

and the organic phase contains

| | |
|---|---|
| aromatic hydrocarbon | 0.5—3% preferably 1% |
| nitrated aromatic hydrocarbon | 97—99.5% preferably 99% |

The temperature of the reaction mass is held at 120—140°C, preferably about 130°C, by heater 4 and is kept under a pressure of about 3450 pascals (gauge).

The inert gas is continuously fed into the reactor through line 5. The gas is fed into the reactor at a temperature of 20—60°C, preferably about 40°C, and is bubbled through the medium by sparger 6 or other suitable means at a rate of 130—150 moles per hour, preferably 145 moles per hour. The gas rises through the reaction mass, is humidified by water extracted from the mass, and is then withdrawn from the reactor through line 7. At this point the gas stream contains, besides water, (a) nitrated product, (b) unreacted hydrocarbon and (c) unreacted nitric acid. Most of (a), (b) and (c) can be extracted from the gas stream by condenser 8, and the dried gas can be fed back to the reactor through line 5.

The condensate from the gas stream comprises water and an organic phase. If desired, this condensate can be fed via line 9 to separator 10, where the water and organic phase are separated. The water can be discarded through line 11, and the organic phase, which contains nitrated hydrocarbon and unreacted hydrocarbon, can be recycled to the reactor through line 12.

The reaction mass is continuously withdrawn from the reactor through line 13. At this point, the mass consists of an aqueous phase, mostly sulfuric acid and water, and an organic phase, principally nitrated hydrocarbon, with minor amounts of unreacted hydrocarbon and reaction byproducts. These phases can be separated in separator 14. The organic phase can be withdrawn through line 15 and conventionally purified to yield nitrated hydrocarbon product. The aqueous phase, mostly sulfuric acid and water, can be recycled to the reactor via line 16.

The following specific Example is intended further to illustrate the invention. In the Example all parts are by weight.

### Example

Benzene is nitrated using apparatus as described above with reference to the drawing.

Reactor 1 is charged with 26,600 parts of 70% sulfuric acid and 20,000 parts of nitrobenzene. Heat is applied with heater 4 and nitrogen is run into the charge through line 5 and sparger 6 at the rate of 2.3 cubic meters per minute (14.5 moles per hour) until the temperature of the charge is 130°C. The nitrogen flow is then increased to 23 cubic meters per minute (145 moles per hour) and the benzene and nitric acid feeds are started and held at rates of 3244 and 4122 parts per hour, respectively.

The gas stream, taken from the reactor through line 7, consists of nitrogen and 20% by weight of water, and a balance made up of nitrobenzene, benzene and nitric acid. The gas stream is fed to condenser 8; the dried gas is fed back to the reactor through line 5. The condensate is fed via line 9 to, and separated in, separator 10; the aqueous phase is discarded via line 11 and the organic phase is recycled through line 12.

The reaction medium, consisting of:—

| | |
|---|---|
| nitrobenzene | 5000 parts |
| benzene | 50.5 |
| dinitrophenol | 17.3 |
| water | 2370 |
| sulfuric acid | 4452 |
| nitric acid | 23.7 |

is continuously withdrawn from the reactor through line 13 and is then passed through separator 14. The aqueous phase is recycled to the reactor via line 16 and the organic phase is withdrawn via line 15 and conventionally purified to give product nitrobenzene.

### Claims

1. A process for continuous nitration of a nitratable aromatic compound by the mixed acid method wherein the reaction mass is contacted with a gas inert to the nitration reaction and capable of being humidified under the reaction conditions, thereby removing water from the reaction mass and withdrawing humidified inert gas from the reaction zone, characterised in that the reaction mass, comprising nitric acid sulphuric acid and the nitratable aromatic compound, is maintained at a temperature of 120 to 140°C while contacting the reaction mass intimately with the inert gas by sparging the inert gas through the reaction mass at a sufficient rate that water is removed from the reaction mass at a rate which maintains at least the minimum sulphuric acid concentration required for continuous nitration of the aromatic compound.

2. A process as claimed in claim 1 wherein the inert gas is nitrogen or carbon dioxide.

3. A process as claimed in claim 1 or claim 2 wherein the aromatic compound is benzene, toluene or chlorobenzene.

4. A process as claimed in any one of claims 1 to 3 wherein the inert gas is treated after contact with the reaction mass to remove water thereform and wherein the so-treated gas is recycled to the reaction mass.

5. A process as claimed in any preceding claim wherein the inert gas at a temperature of from 20°C to 60°C is contacted with the reaction mass.

6. A process as claimed in any preceding claim wherein the sulphuric acid concentration of the reaction mass is maintained at from 70% to 75% by weight of the reaction mass.

**Revendications**

1. Un procédé de nitration continue d'un composé aromatique pouvant être nitré par le procédé aux acides mélangés dans lequel on met en contact la masse réactionnelle avec un gaz inerte vis-à-vis de la réaction de nitration et capable de s'humidifier dans les conditions de réaction, éliminant ainsi de l'eau de la masse réactionnelle, et on retire le gaz inerte humidifié de la zone de réaction, caractérisé en ce qu'on maintient la masse réactionnelle, comprenant de l'acide nitrique, de l'acide sulfurique et le composé aromatique pouvant être nitré, à une température de 120 à 140°C tandis qu'on met en contact intime la masse réactionnelle et le gaz inerte en faisant passer le gaz inerte à travers la masse réactionnelle à un débit suffisant pour que de l'eau soit éliminée de la masse réactionnelle à un débit qui maintienne au moins la concentration minimale en acide sulfurique requise pour la nitration continue du composé aromatique.

2. Un procédé selon la revendication 1, dans lequel le gaz inerte est de l'azote ou du gaz carbonique.

3. Un procédé selon la revendication 1 ou 2, dans lequel le composé aromatique est du benzène, du toluène ou du chlorobenzène.

4. Un procédé selon l'une quelconque des revendications 1 à 3, dans lequel on traite le gaz inerte, après sa mise en contact avec la masse réactionnelle, afin d'en éliminer l'eau, et dans lequel on recycle le gaz ainsi traité à la masse réactionnelle.

5. Un procédé selon l'une quelconque des revendications précédentes, dans lequel on met en contact le gaz inerte à une température de 20 à 60°C avec la masse réactionnelle.

6. Un procédé selon l'une quelconque des revendications précédentes, dans lequel la concentration en acide sulfurique de la masse réactionnelle est maintenue à 70 à 75% du poids de la masse réactionnelle.

**Patentansprüche**

1. Verfahren zur kontinuierlichen Nitrierung einer nitrierbaren aromatischen Verbindung mittels Mischsäure, wobei die Reaktionsmasse mit einem Gas in Kontakt gebracht wird, das gegenüber der Nitrierungsreaktion inert ist und das unter den Reaktionsbedingungen Feuchtigkeit aufnehmen kann, um dadurch Wasser aus der Reaktionsmasse zu entfernen, und das feuchte Inertgas aus der Reaktionsmasse abgezogen wird, dadurch gekennzeichnet, dass die Reaktionsmasse, enthaltend Salpetersäure Schwefelsäure und die nitrierbare aromatische Verbindung, auf einer Temperatur von 120 bis 140°C gehalten wird, während die Reaktionsmasse in engem Kontakt mit dem Inertgas gehalten wird, das durch die Rekationsmasse mit genügender Geschwindigkeit hindurchgeblasen wird, dass Wasser aus der Reactionsmasse mit einer Geschwindigkeit entfernt wird, welche mindestens die für die kontinuierliche Nitrierung der aromatischen Verbindung notwendige Mindestmenge an Schwefelsäure aufrechterhält.

2. Verfahren nach Anspruch 1, worin das Inertgas Stickstoff oder Kohlendioxid ist.

3. Verfahren nach Anspruch 1 oder Anspruch 2, worin die aromatische Verbindung Benzol, Toluol oder Chlorobenzol ist.

4. Verfahren nach einem der Ansprüche 1 bis 3, worin das Inertgas nach dem Kontakt mit der Reaktionsmasse behandelt wird, um Wasser aus ihm zu entfernen, worauf das so behandelte Gas im Kreislauf in die Reaktionsmasse zurückgeführt wird.

5. Verfahren nach einem der vorhergehenden Ansprüche, worin das Inertgas bei einer Temperatur von 20°C bis 60°C mit der Reaktionsmasse kontaktiert wird.

6. Verfahren nach einem der vorhergehenden Ansprüche, worin die Schwefelsäurekonzentration der Reaktionsmasse auf 70 bis 75 Gew.-% der Reaktionsmasse gehalten wird.

1